(19) Europäisches Patentamt
European Patent Office
Office européen des brevets

(11) Publication number: **0 278 319 B1**

(12) **EUROPEAN PATENT SPECIFICATION**

(45) Date of publication of patent specification: **07.10.92**

(51) Int. Cl.$^5$: **C07J 71/00**

(21) Application number: **88101102.7**

(22) Date of filing: **10.06.85**

(60) Publication number of the earlier application in accordance with Art.76 EPC: **0 165 037**

# (54) 9,11-Epoxy-21-hydroxypregnane derivatives.

(30) Priority: **11.06.84 US 618986**
**18.04.85 US 724380**

(43) Date of publication of application:
**17.08.88 Bulletin 88/33**

(45) Publication of the grant of the patent:
**07.10.92 Bulletin 92/41**

(84) Designated Contracting States:
**BE CH DE FR GB IT LI NL SE**

(56) References cited:
**FR-A- 2 318 647**
**GB-A- 2 001 990**
**US-A- 3 210 341**

(73) Proprietor: **THE UPJOHN COMPANY**
**301 Henrietta Street**
**Kalamazoo, Michigan 49001(US)**

(72) Inventor: **VanRheenen, Verlan Henry**
**The Upjohn Company 301 Henrietta Street**
**Kalamazoo Michigan 49001(US)**
Inventor: **Huber, Joel Edward**
**The Upjohn Company 301 Henrietta Street**
**Kalamazoo Michigan 49001(US)**

(74) Representative: **Perry, Robert Edward et al**
**GILL JENNINGS & EVERY 53-64 Chancery Lane**
**London WC2A 1HN(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 278 319 B1

## Description

This invention relates to two novel 9,11-epoxy-21-hydroxypregnane derivatives.

US-A-3210341 (Example 9b) discloses 9β,11β-epoxy-6α-fluoro-21-hydroxypregna-1,4,16-triene-3,20-dione 21-acetate.

The conjugate addition of a methylating agent, such as a methyl Grignard reagent, to a 16-unsaturated steroid to give the corresponding 16α-methyl steroid is well known; see Organic Reactions in Steroid Chemistry, Vol. II, J. Fried and J. A. Edwards, p 75, and US-A-3072686.

The novel compounds of the invention are 9β,11β-epoxy-21-hydroxypregna-4,16-diene-3,20-dione 21-acetate and 9β,11β-epoxy-21-hydroxypregna-1,4,16-triene-3,20-dione 21-acetate.

The first novel compound, mp 129-130.5 °C, is obtained by the general procedure of US-A-3876633, making non-critical variations, but starting with 21-hydroxypregna-1,4,9(11),16-tetraene-3,20-dione 21-acetate (see US-A-2773080).

The second novel compound, mp 163.5-165 °C, is also obtained by the general procedure of US-A-3876633, making non-critical variations, but starting with 21-hydroxypregna-1,4,9(11),16-tetraene-3,20-dione 21-acetate (see US-A-2864834).

The two novel compounds are embodiments of formula I in EP-A-0165037, and are of utility in the preparation of the compounds described and claimed in EP-A-0165037.

Thus, a compound of the invention is reacted with a methylating agent, followed by a silylating agent, to give an enol silane of the formula

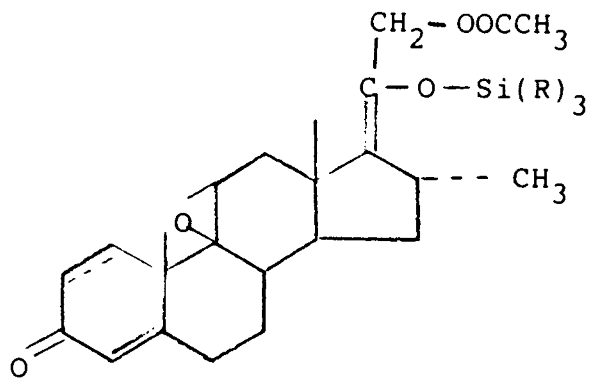

wherein ---- indicates a single or double bond, and each R is independently selected from $C_{1-4}$ alkyl and phenyl.

The methylating agent is selected from $CH_3Cu$, $(CH_3)_2CuM$ wherein M is lithium or magnesium, $CH_3MgQ$ wherein Q is Cl, Br or I, and (in a catalytic amount) copper (cupric) salts. The preferred methylating agent is a methyl Grignard, preferably methylmagnesium chloride. The copper salt can be a cuprous salt such as cuprous chloride, bromide, iodide or cyanide or a cupric salt such as cupric chloride, cupric acetate, cupric proprionate or a complex thereof. Examples of copper complexes include cuprous bromide dimethylsulfide, cuprous chloride tris-n-butylphosphine and cuprous acetylacetonate. The nature of the copper complex is not critical. Hundreds (or thousands) of copper complexes are known which are considered equivalent to those given above. Cupric acetate or propionate is preferred. Additional catalysts which are considered equivalent to those disclosed above are well known to those skilled in the art; see, for example, Alfa Catalog, 1983-1984, Morton Thiokol, Inc., Alpha Products, PO Box 299, 152 Andover Street, Danvers, Mass. 01923, U.S.A.

Solvents suitable for the methylation reaction include tetrahydrofuran, t-butyl methyl ether and dimethoxyethane. The reaction is performed at -50 to 20 °C, preferably at about -20 °C. When TLC indicates that no starting material is left (indicating the probable formation of an enolate intermediate), the silating agent is added and the resulting product is the enol silane. After the silylating agent is added, the reaction temperature is kept at -25 to 25 °C, preferably about 0 °C.

Suitable silylating agents include bistrimethylsilylacetamide and, preferably, those of the formula (R)3-Si-E (wherein E is Cl, Br, I or $NR_\alpha R_\beta$ in which either $R_\alpha$ and $R_\beta$ are independently selected from $C_{1-5}$ alkyl or phenyl or are connected to form a ring with or without an oxygen atom or an additional nitrogen atom), more preferably trimethylsilyl chloride. Additional silylating agents considered equivalent to those disclosed above are well known to those skilled in the art; see, for example Silicon Compounds, Petrarch Systems,

Inc., Bartram Rd. Bristol, PA 19007. The trapping of an enolate intermediate to produce a $\Delta^{17(20)}$-20-(acetate) is known; see US-A-4031080 and Organic Reactions in Steroid Chemistry, Vol. II, supra, p 76. Known enol acylates are too unreactive to react selectively over A,B,C-ring functionality such as $A^{9(11)}$ However, the enol silanes of the given formula are, surprisingly and unexpectedly, sufficiently reactive to react with electrophiles without affecting most of the other functionality in the A,B,C-rings.

By way of example, a mixture of 9β,11β-epoxy-21-hydroxypregna-1,4,16-triene-3,20-dione 21-acetate (7.650 and cupric acetate monohydrate (400 g) in dry THF (130 ml) is stirred under nitrogen. The temperature is adjusted to -20° C and 1,1,3,3-tetramethylurea (5.0 ml) is added. Methylmagnesium chloride (2 M, 16.6 ml) is added dropwise over 50 min at -17 to -19° C. TLC indicates the Grignard reaction is complete. The mixture is stirred for 40 min at -19° C, following which the magnesium enolate is quenched with trimethylchlorosilane (3.28 ml). The temperature rises to 25° C during the 1 hr stir period. TLC shows this reaction is complete. The mixture is added to toluene (150 ml) and monobasic potassium phosphate buffer (pH 4.3, 10%, 50 ml). The aqueous layer is extracted with toluene (25 ml) and then is discarded. The two organic layers are washed sequentially with water (2 x 50 ml), then combined and concentrated to a high boiling residue containing the $\Delta^{17(20)}$-20-enol silane.

## Claims

1. The compound 9β,11β-epoxy-21-hydroxypregna-4,16-diene-3,20-dione 21-acetate or 9β,11β-epoxy-21-hydroxypregna-1,4,16-triene-3,20-dione 21-acetate.

2. A process for preparing 9β,11β-epoxy-20,21-dihydroxy-16α-methylpregna-4,17(20)-dien-3-one 21-acetate or 9β,11β-epoxy-20,21-dihydroxy-16α-methylpregna-1,4,17(20)-trien-3-one 21-acetate, which comprises reacting a respective compound of claim 1 with a methylating agent; and trapping the product as the corresponding 20-$Si(R)_3$-substituted enol silane, wherein each R is independently selected from $C_{1-4}$ alkyl and phenyl, by reaction with a suitable silylating agent.

3. A process according to claim 2, wherein the methylating agent is $CH_3Cu$, $(CH_3)_2CuM$ or $CH_3MgQ$, M being Li or Mg and Q being Cl, Br or I.

## Patentansprüche

1. Die Verbindung 9β,11β-Epoxy-21-hydroxypregna-4,16-dien-3,20-dion-21-acetat oder 9β,11β-Epoxy-21-hydroxypregna-1,4,16-trien-3,20-dion-21-acetat.

2. Verfahren zur Herstellung von 9β,11β-Epoxy-20,21-dihydroxy-16α-methylpregna-4,17(20)-dien-3-on-21-acetat oder 9β,11β-Epoxy-20,21-dihydroxy-16α-methylpregna-1,4,17(20)-trien-3-on-21-acetat durch Umsetzen der entsprechenden Verbindung von Anspruch 1 mit einem Methylierungsmittel und Abfangen des Produkts in Form des entsprechenden 20-$Si(R)_3$-substituiertenEnolsilans, worin jeder Rest R unabhängig für $C_{1-4}$-Alkyl oder Phenyl steht, durch Umsetzen mit einem geeigneten Silylierungsmittel.

3. Verfahren nach Anspruch 2, worin das Methylierungsmittel aus $CH_3Cu$, $(CH_3)_2CuM$ mit M gleich Li oder Mg, oder $CH_3MgQ$ mit Q gleich Cl, Br oder I, besteht.

## Revendications

1. Le 21-acétate de 9β,11β-époxy-21-hydroxyprégna-4,16-diène-3,20-dione ou 21-acétate de 9β,11β-époxy-21-hydroxyprégna-1,4,16-triène-3,20-dione.

2. Procédé de préparation de 21-acétate de 9β,11β-époxy-20,21-dihydroxy-16α-méthylprégna-4,17(20)-diène-3-one ou de 21-acétate de 9β,11β-époxy-20,21-dihydroxy-16α-méthylprégna-1,4,17(20)-triène-3-one, qui consiste à faire réagir l'un des composés suivant la revendication 1 avec un agent de méthylation ; et à piéger le produit sous forme de l'énolsilane correspondant substitué avec un groupe 20-$Si(R)_3$, dans lequel chaque groupe R est choisi indépendamment entre des groupes alkyle en $C_1$ à $C_4$ et phényle, par réaction avec un agent de silylation convenable.

3. Procédé suivant la revendication 2, dans lequel l'agent de méthylation est $CH_3Cu$, $(CH_3)_2CuM$ ou $CH_3MgQ$, M représentant Li ou Mg et Q représentant Cl, Br ou I.